(19) Europäisches Patentamt
European Patent Office
Office européen des brevets

(11) EP 0 584 720 B1

(12) **EUROPÄISCHE PATENTSCHRIFT**

(45) Veröffentlichungstag und Bekanntmachung des Hinweises auf die Patenterteilung:
**14.05.1997 Patentblatt 1997/20**

(51) Int Cl.6: **C07F 15/00**, C07C 45/50

(21) Anmeldenummer: **93113258.3**

(22) Anmeldetag: **19.08.1993**

(54) **Verfahren zur Wiedergewinnung von Rhodium aus den Destillationsrückständen von Produkten der Oxosynthese**

Process for the recovery of rhodium from distillation residues from products of the oxosynthesis

Procédé de récupération de rhodium à partir de résidus de distillation de l'oxosynthèse

(84) Benannte Vertragsstaaten:
**AT BE DE ES FR GB IT NL SE**

(30) Priorität: **28.08.1992 DE 4228724**

(43) Veröffentlichungstag der Anmeldung:
**02.03.1994 Patentblatt 1994/09**

(73) Patentinhaber: HOECHST
**AKTIENGESELLSCHAFT**
**65926 Frankfurt am Main (DE)**

(72) Erfinder:
• **Diekhaus, Gerhard, Dr.Dipl.-Chem.**
  **D-4200 Oberhausen 11 (DE)**
• **Kappesser, Harald**
  **D-4200 Oberhausen 11 (DE)**

(56) Entgegenhaltungen:
EP-A- 0 147 824         EP-A- 0 348 833
EP-A- 0 354 588         EP-A- 0 424 736
DE-A- 2 627 354

## Beschreibung

Die vorliegende Erfindung betrifft ein verbessertes Verfahren zur Wiedergewinnung von Rhodium aus den Destillationsrückständen von Produkten der Oxosynthese.

Die Herstellung von Aldehyden und Alkoholen durch katalytische Anlagerung von Kohlenmonoxid und Wasserstoff an olefinische Doppelbindungen (Hydroformylierung) ist bekannt. Moderne Verfahren arbeiten mit metallischem Rhodium oder mit Rhodiumverbindungen als Katalysatoren, die allein oder in Kombination mit komplexbildenden Liganden, z.B. organischen Phosphinen oder Estern der phosphorigen Säure, eingesetzt werden. Unter den Reaktionsbedingungen als Katalysator wirksam sind nach übereinstimmender Auffassung der Fachwelt Hydridocarbonylverbindungen des Rhodiums, die sich durch die allgemeine Formel $H[Rh(CO)_{4-x}L_x]$ wiedergeben lassen, wobei L einen Liganden bezeichnet und x 0 oder eine ganze Zahl von 1 bis 3 ist.

Die Verwendung von Rhodiumkatalysatoren hat gegenüber der klassischen Oxosynthese mit Kobaltkatalysatoren eine Reihe Vorteile. Die Aktivität von Rhodiumkatalysatoren ist höher als die von Kobaltkatalysatoren und endständige Olefine werden in Gegenwart von Rhodium (in Form von Rhodium-Komplexverbindungen) in höherem Maße zu unverzweigten Aldehyden umgesetzt, als in Gegenwart von Kobalt. Überdies lassen sich Produktionsanlagen bei Verwendung von Rhodiumkatalysatoren weitgehend problemlos betreiben, das betrifft insbesondere die Durchführung der Synthese und die Ausbringung der Produkte.

Ein für die Wirtschaftlichkeit des Rhodiumverfahrens bestimmender Faktor ist die möglichst verlustfreie Abtrennung und Wiedergewinnung des Edelmetalls, unabhängig davon, ob es mit oder ohne zusätzlichem Komplexbildner als Katalysator eingesetzt wurde. Nach Beendigung der Umsetzung findet sich das Rhodium als Carbonylverbindung, die gegebenenfalls noch weitere Liganden enthält, gelöst im Hydroformylierungsprodukt.

Zur Aufarbeitung wird das Rohprodukt der Synthese unter Freisetzen gelösten Synthesegases zunächst ein- oder mehrstufig auf Normaldruck entspannt. Die Abtrennung des Rhodiums erfolgt entweder unmittelbar aus dem entspannten Rohprodukt oder aus dem Rückstand der Rohproduktdestillation. Der erste Weg wird dann beschritten, wenn in der vorausgegangenen Hydroformylierungsstufe Rhodium ohne zusätzlichen Komplexbildner als Katalyator eingesetzt worden war. Die zweite Variante wird angewandt, wenn der Rhodiumkatalysator außer Kohlenmonoxid noch weitere Liganden, z.B. Phosphine oder Phosphite in komplexer Bindung enthält. Sie kann auch dann genutzt werden, wenn die Hydroformylierung zwar mit Rhodium allein durchgeführt, dem Rohprodukt nach Entspannung aber ein Komplexbildner zur Stabilisierung des Rhodiums zugesetzt worden war oder in anderer Weise, z.B. durch Destillation unter Druck dafür Sorge getragen wird, daß das Rhodium nicht in Form flüchtiger Verbindungen aus dem Destillationsgut oder dem Destillationsrückstand entweicht.

Unabhängig von der gewählten Form der Aufbereitung des Reaktionsgemischs ist zu berücksichtigen, daß das Edelmetall im Rohprodukt in einer Konzentration von nur wenigen ppm vorliegt, seine Abtrennung daher sehr umsichtiges Arbeiten erfordert. Zusätzliche Schwierigkeiten können auch dadurch entstehen, daß das Rhodium, insbesondere wenn es ohne Ligand eingesetzt wurde, bei der Entspannung teilweise in metallische Form übergeht oder mehrkernige Carbonyle bildet. Es kommt dann zur Ausbildung eines heterogenen Systems, das aus der flüssigen organischen und der festen, Rhodium oder Rhodiumverbindungen enthaltenden Phase besteht.

Unter diesen Umständen überrascht es nicht, daß die Rückgewinnung von Rhodium aus den Produkten der Oxysynthese, darunter auch den Rückständen von Oxorohprodukten, vielfach untersucht wurde. Die Arbeiten führten zur Entwicklung zahlreicher Verfahren, von denen einige auch Anwendung im technischen Maßstab gefunden haben.

Nach einem in der Praxis bewährten Verfahren zur Wiedergewinnung von Rhodium, das in komplexer Bindung mit einer organischen Phosphor(III)-Verbindung in den Rückständen der Destillation von Produkten der Oxosynthese enthalten ist, behandelt man die Rückstände mit Sauerstoff oder einem Sauerstoff enthaltenden Gas bei 60 bis 120°C drucklos oder unter Druck in Gegenwart einer $C_2$- bis $C_5$-Monocarbonsäure und des Alkalisalzes einer $C_2$- bis $C_5$-Monocarbonsäure und extrahiert darauf die Rhodiumverbindung mit Wasser (vgl. EP 424 736 Al).

Gegenstand der US-Patentschrift 42 92 196 ist die Extraktion von Metallen der 8. Gruppe, die als Katalysatoren in Form von Metallcarbonylen oder metallorganischen Verbindungen homogen gelöst in Hydroformylierungsprodukten vorliegen, mit wasserlöslichen, Stickstoff enthaltenden Verbindungen. Die Abtrennung erfolgt bei Temperaturen, die zwischen Raumtemperatur und 100°C liegen und in einem Bereich von normalem bis zu 7 MPa erhöhtem Druck. Als stickstoffhaltige Verbindungen werden zur Extraktion Ammoniak, Ammoniumhydroxid und Amine eingesetzt.

Ein weiteres Verfahren zur Abtrennung von Rhodium aus den Produkten der Oxosynthese, das auf der Extraktion mit einem komplexbildenden Reagens beruht, ist in der EP 147 824 B1 beschrieben. Als komplexbildende Reagenzien werden wasserlösliche Sulfonate oder Carboxylate organischer Phosphine in Form einer wäßrigen Lösung, die mit dem Oxorohprodukt nicht mischbar ist, eingesetzt.

In den beiden zuletzt beschriebenen Fällen werden die in den Hydroformylierungsprodukten vorliegenden Metallcarbonyl- oder metallorganischen Verbindungen ohne vorherige Aufspaltung der koordinativen oder der Metall-Kohlenstoff-Bindungen mit dem Extraktionsmittel behandelt.

Die bekannten Verfahren erlauben es, bei technischer Durchführung bis zu 90 % des ursprünglich eingesetzten Rhodiums wiederzugewinnen, der Rest des Edelmetalls geht verloren. Probleme bei der Aufarbeitung der Rhodiumextrakte treten bisweilen dadurch auf, daß die Rhodiumkonzentration in den wäßrigen Lösungen gering ist und entweder große Flüssigkeitsvolumina behandelt oder die Lösungen zuvor konzentriert werden müssen. Daher besteht Interesse daran, die Wiedergewinnung des Rhodiums aus den Produkten der Oxosynthese zu vervollkommnen, die Rhodiumverluste weiter zu verringern und die Handhabung der Extrakte zu vereinfachen.

Die Erfindung löst die vorstehend beschriebene Aufgabe durch ein Verfahren zur Wiedergewinnung von Rhodium, das, gegebenenfalls in komplexer Bindung, im Destillationsrückstand von Produkten der Oxosynthese enthalten ist, durch Behandlung des Rückstandes mit Sauerstoff oder einem Sauerstoff enthaltenden Gas bei 60 bis 120°C, drucklos oder unter Druck in Gegenwart einer $C_2$- bis $C_5$-Monocarbonsäure und des Alkalisalzes einer $C_2$- bis $C_5$-Monocarbonsäure und anschließende Extraktion des als wasserlösliche Verbindung vorliegenden Rhodiums. Es ist dadurch gekennzeichnet, daß der Rückstand mit der wäßrigen Lösung eines mit Rhodium komplexbildenden Reagens extrahiert wird, gegebenenfalls nachdem er zuvor bereits mit Wasser allein extrahiert worden war.

Das neue Verfahren stellt sicher, daß ein sehr hoher Anteil des im Oxorohprodukt enthaltenden Rhodiums abgetrennt wird. Mit seiner Hilfe gelingt es, Rhodium auch dann zurückzugewinnen, wenn z.B. bei Vorliegen sehr niedriger Metallkonzentrationen Wasser allein nicht oder nur wenig wirksam ist. Dabei erhält man das Rhodium wahlweise überwiegend als leicht weiterzuverarbeitende, binäre Verbindung oder aber ausschließlich als katalytisch wirksame, wasserlösliche Komplexverbindung. Besonders hervorzuheben ist in diesem Zusammenhang, daß die Extraktionslösung Rhodium in einer Konzentration enthält, die ihre Aufarbeitung oder ihren Wiedereinsatz problemlos ermöglicht.

Das neue Verfahren geht von den Rückständen der Hydroformylierung olefinisch ungesättigter Verbindungen aus, die nach dem Abdestillieren der Aldehyde und Alkohole als Destillationssumpf anfallen. Sie bestehen im wesentlichen aus höhermolekularen Verbindungen, die aus den Aldehyden durch Aldolkondensation entstanden sind und in einer Folgereaktion unter Bildung ungesättigter Verbindungen auch Wasser abspalten können. Die Art der Verbindungen, die hydroformyliert wurden, ist für die beanspruchte Arbeitsweise ohne Bedeutung. Dementsprechend können sowohl Rückstände eingesetzt werden, die aus der Reaktion von Olefinen mit Kohlenmonoxid und Wasserstoff resultieren, als auch Rückstände, die bei der Umsetzung olefinisch ungesättigter Verbindungen entstehen, die noch funktionelle Gruppen im Molekül enthalten. Im Vordergrund des neuen Verfahrens steht die Wiedergewinnung von Rhodium aus den Rückständen der Hydroformylierung von Olefinen mit 2 bis 12 Kohlenstoffatomen, entsprechend der wirtschaftlichen Bedeutung der aus ihnen hergestellten Aldehyde. Neben Aldehyden und Alkoholen und den gesättigten und ungesättigten Kondensationsprodukten können die zu verarbeitenden Gemische als wesentliche Bestandteile auch noch Lösungsmittel und ferner Verbindungen enthalten, die mit Rhodiumbzw. Rhodiumcarbonylverbindungen unter Komplexbildung reagieren und gegenüber dem Rhodium zumeist im Überschuß vorhanden sind. Zu diesen Verbindungen gehören organische Phosphor(III)-verbindungen insbesondere Phosphine und Phosphite, vorzugsweise die Arylverbindungen wie Triphenylphosphin und phenylphosphit.

Erfindungsgemäß wird der Destillationsrückstand mit Sauerstoff behandelt, um das als Carbonylverbindung oder in anderer komplexer Bindung vorliegende Rhodium in leicht extrahierbare Form zu überführen. Das Oxidationsmittel wird in reiner Form eingesetzt oder als Sauerstoff enthaltendes Gasgemisch, insbesondere Luft. Die Sauerstoffmenge kann in weiten Grenzen variiert werden. Sie richtet sich nach der Rhodiumkonzentration und nach der Konzentration der Liganden, also vorzugsweise der Phosphor(III)-verbindung im Rückstand. Es empfiehlt sich je mol Rhodium und je mol Ligand 100 bis 2000, insbesondere 300 bis 1200 mol Sauerstoff anzuwenden.

Entsprechend der Erfindung erfolgt die Behandlung des Destillationsrückstandes mit Sauerstoff in Gegenwart einer gesättigten, geradkettigen oder verzweigten Monocarbonsäure mit 2 bis 5 Kohlenstoffatomen. Beispiele für geeignete Säuren sind Essigsäure, Propionsäure, n-Buttersäure, i-Buttersäure und n-Valeriansäure. Besonders bewährt haben sich Essigsäure und Propionsäure. Sie werden in der handelsüblichen Form und in einer solchen Menge eingesetzt, daß je mol Rhodium etwa 2 bis 150, vorzugsweise 3 bis 50 mol Säure vorhanden sind. Die Säure wird dem Gemisch vor der Umsetzung mit Sauerstoff zugesetzt unabhängig davon, daß sich aufgrund vorhandenen Aldehyds im Laufe der Reaktion ebenfalls Säure bilden kann.

Ein weiteres, wichtiges Merkmal des erfindungsgemäßen Verfahrens ist die Anwesenheit eines Alkalicarboxylats im Einsatzmaterial während der Sauerstoffbehandlung. Als Alkalicarboxylate werden im Rahmen des neuen Verfahrens Salze gesättigter, geradkettiger oder verzweigter Monocarbonsäuren mit 2 bis 5 Kohlenstoffatomen verwendet. Besonders bewährt haben sich die Natrium- und Kaliumsalze der Essigsäure, der Propionsäure, der n- und iso-Buttersäure und der n-Valeriansäure. Sie werden in einer Menge von 10 bis 250, vorzugsweise 20 bis 180 mol je mol Rhodium angewandt. Geeignet sind die handelsüblichen Salze, die jedoch erst im Verlauf der Oxidation allmählich in Lösung gehen. Vorteilhafter ist es daher, zum Rückstand freie Säure und die äquivalente Menge Alkalihydroxid zu geben, die sofort homogen gelöst und damit voll wirksam werden.

Die Umsetzung mit Sauerstoff erfolgt bei 60 bis 120, vorzugsweise 80 bis 100°C. Sie kann drucklos oder unter Druck vorgenommen werden, Drücke zwischen 0,2 und 1,0 MPa haben sich besonders bewährt.

Die Reaktionszeit ist abhängig von der Rhodium- und der Ligandenkonzentration im Einsatzmaterial. Sie wird weiterhin durch die eingesetzte Sauerstoffmenge, durch tionstemperatur und -druck bestimmt. Hohe Konzentrationen der gelösten Substanzen erfordern längere Behandlungszeiten als niedrige Konzentrationen. Ein großes Sauerstoffangebot und erhöhter Druck vermindern die Reaktionszeit ebenso wie eine intensive Durchmischung des Rückstandes mit dem Sauerstoff. Temperaturen im unteren und im oberen Bereich des beanspruchten Intervalls sind etwas weniger effektiv als im mittleren Temperaturbereich.

Die Umsetzung der Destillationsrückstände kann in konventionellen Apparaturen kontinuierlich oder diskontinuierlich durchgeführt werden. Der Sauerstoff oder das Sauerstoff enthaltende Gas wird über Verteilungsvorrichtungen in den Reaktor geleitet, die gleichmäßige Vermischung von flüssiger und gasförmiger Phase gegebenenfalls durch Rühren unterstützt.

Nach Abschluß der Behandlung mit Sauerstoff wird die organische Phase mit der wäßrigen Lösung eines Reagens, das Rhodium komplex bindet, extrahiert. Als komplexbildende Reagenzien werden erfindungsgemäß Verbindungen bezeichnet, die unter den herrschenden Temperatur- und Druckbedingungen mit dem Rhodium stabile, in Wasser lösliche Komplexverbindungen eingehen. Je größer die Neigung der komplexbildenden Reagenzien zur Reaktion mit dem Rhodium ist, desto vollständiger ist die Abtrennung des Edelmetalls aus dem mit Sauerstoff vorbehandelten Rohprodukt oder Destillationsrückstand. Die Komplexbildner können ein- oder mehrzähnig sein, also eine oder mehrere Koordinationsstellen des Zentralatoms besetzen.

Bevorzugte Komplexbildner für das Rhodium sind Verbindungen des Stickstoffs und des Phosphors, die zur Ausbildung von Koordinationsbindungen mit dem Rhodium befähigte Stickstoff- bzw. Phosphoratome enthalten, d.h. Atome, die über freie Elektronenpaare verfügen. Beispiele für Stickstoffverbindungen sind Ammoniak, wasserlösliche primäre, sekundäre oder tertiäre Amine und Diamine, Alkoholamine, Aminocarbonsäuren. Als Phosphorverbindungen kommen insbesondere sulfonierte oder carboxylierte Arylphosphine oder Aryldiphosphine in Betracht. Bevorzugt werden Iminodiessigsäure, Nitrilotriessigsäure, diamintetraessigsäure, Triphenylphosphintrisulfonate und Triphenylphosphindisulfonate der Alkalimetalle und des Ammoniums.

Bezogen auf Rhodium werden die komplexbildenden Reagenzien im Überschuß angewendet. Da man sie im Kreis führen kann, ist die Höhe des Überschusses an sich beliebig, jedoch sollten je mol Rhodium mindestens 5 mol eines einzähnigen Komplexbildners oder mindestens $\frac{5}{n}$ mol eines n-zähnigen Komplexbildners vorhanden sein. Bewährt hat es sich, je mol Rhodium 10 bis 50 mol eines einzähnigen Komplexbildners bzw. $\frac{10}{n}$ bis $\frac{50}{n}$ mol eines n-zähnigen Komplexbildners anzuwenden.

Die Konzentration des komplexbildenden Reagenzes im Lösungsmittel ist in weiten Grenzen variabel. Sie hängt insbesondere davon ab, in welchem Maße das Rhodium angereichert werden soll. Dementsprechend können nicht nur stark verdünnte, sondern gegebenenfalls sogar gesättigte Lösungen Anwendung finden. In der Regel setzt man Lösungen ein, die 0,5 bis 25 Gew.-% des Komplexbildners, bezogen auf die Lösung, enthalten.

Die Extraktion des Rhodiums mit dem gelösten Komplexbildner wird bei Temperaturen von 20 bis 120°C durchgeführt. Die Abtrennung kann bei normalem Druck erfolgen, aber auch bei erhöhten Drücken bis zu 1,0 MPa, bevorzugt ist der Bereich von 0,2 bis 1,0 MPa.

In vielen Fällen genügt eine einmalige Behandlung des Destillationsrückstandes mit der Lösung des komplexbildenden Reagens. Selbstverständlich kann die Lösung rezirkuliert werden, um durch mehrfache Behandlung der organischen Phase die Rhodiumabtrennung zu vervollständigen und die Rhodiumkonzentration im Extraktionsmittel zu erhöhen. Auch eine mehrstufige Extraktion ist möglich. Der Extraktion mit der wäßrigen Lösung eines komplexbildenden Reagens kann eine Extraktion mit Wasser vorausgehen. Sie wird üblicherweise mit entionisiertem Wasser bei 20 bis 120°C und drucklos oder bei Drücken bis 1,0 MPa, vorzugsweise 0,2 bis 1,0 MPa, insbesondere in mehreren Stufen, durchgeführt. Die eingesetzte Wassermenge richtet sich nach dem Verteilungsgleichgewicht der zu extrahierenden Substanz zwischen organischer und wäßriger Phase und der angestrebten Rhodiumkonzentration in der wäßrigen Phase. Die wäßrige Extraktionslösung kann bis zur Einstellung des Verteilungsgleichgewichts im Kreis geführt und dadurch wiederholt zur Abtrennung von Rhodium verwendet werden, um eine Anreicherung des Metalles in der Lösung zu erzielen. Im allgemeinen werden zwei bis drei Extraktionsstufen vorgesehen. Der Übergang von dem einen Extraktionsmittel auf das andere ist den individuellen Gegebenheiten anzupassen. Maßgebend sind insbesondere das Nachlassen der Wirksamkeit des reinen Wassers zur Rhodiumabtrennung, erkennbar an der je Volumeneinheit Wasser aufgenommenen Rhodiummenge und die angestrebte Rhodiumkonzentration in der wäßrigen Extraktionslösung.

Der neue Prozeß kann absatzweise oder kontinuierlich in den für die Solventextraktion üblichen Reaktoren durchgeführt werden. Die Extraktion kann sowohl im Gleichstrom als auch im Gegenstrom erfolgen.

Die Weiterbehandlung oder Weiterverwendung der das abgetrennte Rhodium enthaltenden Phasen richtet sich nach den jeweiligen Gegebenheiten. So kann das als binäre Verbindung extrahierte Rhodium aus einer wäßrigen Lösung als Salz einer höheren Carbonsäure abgeschieden werden. Ebenso ist es möglich, die wäßrige Lösung der binären Rhodiumverbindung, z.B. nach Zusatz eines wasserlöslichen Phosphins, wieder als Katalysatorphase in der Oxosynthese einzusetzen. Die komplexgebundenes Rhodium enthaltende Wasserphase wird vorzugsweise als Katalysator verwendet, gegebenenfalls nach Zusatz weiterer Rhodiums und/ oder weiterer Komplexbildner.

In den nachfolgenden Beispielen wird die Erfindung erläutert. Es ist aber nicht beabsichtigt, sie auf diese speziellen Ausführungsformen zu beschränken.

Beispiel 1

In einem Rührwerksreaktor werden 90,3 kg eines Destillationsrückstandes der Ethylen-Hydroformylierung mit soviel Toluol verdünnt, daß die Rhodiumkonzentration in der Lösung 70 ppm beträgt. Man gibt 534 g Na-propionat und 55 g Propionsäure hinzu und leitet bei 80°C unter einem Druck von 0,25 MPa während 6 h 120m$^3$ Luft durch den gerührten Reaktorinhalt. Anschließend kühlt man auf unterhalb 60°C ab, rührt das Gemisch drucklos 15 min mit 25 l Wasser und scheidet wäßrige und organische Phase voneinander. Diese Behandlung wird zweimal mit je 10 l Wasser wiederholt. Sie führt zur Abtrennung von insgesamt 83,5 % des ursprünglich in der organischen Phase enthaltenen Rhodiums. Darauf rührt man die organische Phase, sie enthält noch Rhodium in einer Konzentration von 13,5 ppm, drucklos, bei Raumtemperatur 15 min mit einer solchen Menge einer Trinatrium-triphenylphosphin-trisulfonat-Lösung (1 Gew.-% in Wasser) daß je mol Rhodium 32,2 mol des Phosphins vorliegen. Die Menge des abgetrennten Rhodiums erhöht sich durch diesen Extraktionsschritt auf insgesamt 88,8 %, in der organischen Phase ist das Metall noch in einer Konzentration von 10,2 ppm enthalten.

Beispiel 2

In einem Rührwerksbehälter werden 61 kg eines Destillationsrückstandes der Ethylen-Hydroformylierung mit soviel Toluol verdünnt, daß die Rhodiumkonzentration in der Lösung 70 ppm beträgt. Man gibt 655 g Na-propionat und 67 g Propionsäure hinzu und leitet bei 80°C unter einem Druck von 0,25 MPa während 6 h 120m$^3$ Luft durch den gerührten Reaktorinhalt. Anschließend kühlt man auf unterhalb 60°C ab, rührt das Gemisch drucklos 15 min mit 25 l Wasser und scheidet wäßrige und organische Phase voneinander. Diese Behandlung wird dreimal mit je 10 l Wasser wiederholt. Sie führt zur Abtrennung von insgesamt 84,9 % des ursprünglich in der organischen Phase enthaltenen Rhodiums. Darauf rührt man die organische Phase, sie enthält noch Rhodium in einer Konzentration von 12,8 ppm, drucklos, bei Raumtemperatur 15 min mit einer solchen Menge einer Trinatrium-triphenylphosphin-trisulfonat-Lösung (1 Gew.-% in Wasser), daß je mol Rhodium 28,3 mol des Phosphins vorliegen. Die Menge des abgetrennten Rhodiums erhöht sich durch diesen Extraktionsschritt auf insgesamt 91,4 %, in der organischen Phase ist das Metall noch in einer Konzentration von 7,4 ppm enthalten.

Beispiel 3

In einem Rührreaktor werden 150 kg eines Destillationsrückstandes der Ethylen-Hydroformylierung mit soviel Toluol verdünnt, daß die Rhodiumkonzentration in der Lösung 70 ppm beträgt. Man gibt 557 g Na-propionat und 58 g Propionsäure hinzu und leitet bei 80°C unter einem Druck von 0,25 MPa während 6 h 120 m$^3$ Luft durch den gerührten Reaktor. Anschließend kühlt man auf unterhalb 60°C ab, rührt das Gemisch drucklos 15 min mit 25 l Wasser und scheidet wäßrige und organische Phase voneinander. Diese Behandlung wird dreimal mit je 10 l Wasser wiederholt. Sie führt zur Abtrennung von insgesamt 72,7 % des ursprünglich in der organischen Phase enthaltenen Rhodiums. Darauf rührt man die organische Phase, sie enthält noch Rhodium in einer Konzentration von 19,7 ppm, drucklos, bei Raumtemperatur 15 min mit einer solchen Menge einer Ethylendiamin-tetraessigsäure-Lösung (1 Gew.-% in Wasser), daß je mol Rhodium 10,7 mol des Diamins (entsprechend 21,4 mol zur Komplexbildung fähiger Aminstickstoff) vorliegen. Die Menge des abgetrennten Rhodiums erhöht sich durch diesen Extraktionsschritt auf insgesamt 81,7 %, in der organischen Phase ist das Metall noch in einer Konzentration von 14,9 ppm enthalten.

Beispiel 4

In einem Rührreaktor werden 150 kg eines Destillationsrückstandes der Ethylen-Hydroformylierung mit soviel Toluol verdünnt, daß die Rhodiumkonzentration in der Lösung 70 ppm beträgt. Man gibt 557 g Na-propionat und 58 g Propionsäure hinzu und leitet bei 80°C unter einem Druck von 0,25 MPa während 6 h 120 m$^3$ Luft durch den gerührten Reaktorinhalt. Anschließend kühlt man auf unterhalb 60°C ab, rührt das Gemisch drucklos 15 min mit 25 l Wasser und scheidet wäßrige und organische Phase voneinander. Diese Behandlung wird dreimal mit je 10 l Wasser wiederholt. Sie führt zur Abtrennung von insgesamt 76,7 % des ursprünglich in der organischen Phase enthaltenen Rhodiums. Darauf rührt man die organische Phase, sie enthält noch Rhodium in einer Konzentration von 19,7 ppm, drucklos, bei Raumtemperatur 15 min mit einer solchen Menge einer Nitrilotriessigsäure-Lösung (1 Gew.-% in Wasser), daß je mol Rhodium 10,9 mol des Phosphins vorliegen. Die Menge des abgetrennten Rhodiums erhöht sich durch diesen Extraktionsschritt auf insgesamt 83,3 %, in der organischen Phase ist das Metall noch in einer Konzentration von 12,8 ppm enthalten.

Beispiel 5

In einem Rührreaktor werden 70 kg eines Destillationsrückstandes der Acrylsäuremethylester-Hydroformylierung mit soviel Toluol verdünnt, daß die Rhodiumkonzentration in der Lösung 70 ppm beträgt. Man gibt 737 g Na-propionat und 76 g Propionsäure hinzu und leitet bei 100°C unter einem Druck von 0,25 MPa während 6 h 100 m$^3$ Luft durch den gerührten Reaktorinhalt. Anschließend kühlt man auf unterhalb 60°C ab, rührt das Gemisch drucklos 15 min mit 32 l Wasser und scheidet wäßrige und organische Phase voneinander. Diese Behandlung wird einmal mit 10 l Wasser wiederholt. Sie führt zur Abtrennung von insgesamt 81,4 % des ursprünglich in der organischen Phase enthaltenen Rhodiums. Darauf rührt man die organische Phase, sie enthält noch Rhodium in einer Konzentration von 15,5 ppm, drucklos, bei Raumtemperatur 15 min mit einer solchen Menge einer Trinatrium-triphenylphosphintrisulfonat-Lösung (1 Gew.-% in Wasser), daß je mol Rhodium 14,5 mol des Phosphins vorliegen. Die Menge des abgetrennten Rhodiums erhöht sich durch diesen Extraktionsschritt auf insgesamt 84,5 %, in der organischen Phase ist das Metall noch in einer Konzentration von 14,8 ppm enthalten.

Beispiel 6

In einem Rührwerksbehälter werden 140 kg eines Destillationsrückstandes der Acrylsäuremethylester-Hydroformylierung mit soviel Toluol verdünnt, daß die Rhodiumkonzentration in der Lösung 5,5 ppm beträgt. Man gibt 701 g Na-propionat und 77 g Propionsäure hinzu und leitet bei 100°C unter einem Druck von 0,25 MPa während 6 h 100 m$^3$ Luft durch den gerührten Reaktorinhalt. Anschließend kühlt man auf unterhalb 60°C ab, rührt das Gemisch drucklos 15 min mit 10 l Wasser und scheidet wäßrige und organische Phase voneinander. Diese Behandlung führt zur Abtrennung von 67,4 % des ursprünglich in der organischen Phase enthaltenen Rhodiums. Darauf rührt man die organische Phase, sie enthält noch Rhodium in einer Konzentration von 4,3 ppm, drucklos, bei Raumtemperatur 15 min mit einer solchen Menge einer Trinatrium-triphenylphosphintrisulfonat-Lösung, daß je mol Rhodium 45,2 mol des Phosphins vorliegen. Die Menge des abgetrennten Rhodiums erhöht sich durch diesen Extraktionsschritt auf insgesamt 79 %, in der organischen Phase ist das Metall noch in einer Konzentration von 3,6 ppm enthalten.

**Patentansprüche**

1. Verfahren zur Wiedergewinnung von Rhodium, das, gegebenenfalls in komplexer Bindung, im Destillationsrückstand von Produkten der Oxosynthese enthalten ist, durch Behandlung des Rückstandes des mit Sauerstoff oder einem Sauerstoff enthaltenden Gas bei 60 bis 120°C, drucklos oder unter Druck in Gegenwart einer $C_2$- bis $C_5$-Monocarbonsäure und des Alkalisalzes einer $C_2$- bis $C_5$-Monocarbonsäure und anschließende Extraktion des als wasserlösliche Verbindung vorliegenden Rhodiums, dadurch gekennzeichnet, daß der Rückstand mit der wäßrigen Lösung eines mit Rhodium komplexbildenden Reagens extrahiert wird, gegebenenfalls nachdem er zuvor bereits mit Wasser allein extrahiert worden war.

2. Verfahren nach Anspruch 1, dadurch gekennzeichnet, daß die Extraktion mit der Lösung des komplexbildenden Reagens bei Temperaturen von 20 bis 120°C und Drücken bis 1,0 MPa, vorzugsweise von 0,2 bis 1,0 MPa erfolgt.

3. Verfahren nach Anspruch 1, dadurch gekennzeichnet, daß die Extraktion mit Wasser bei Temperaturen von 20 bis 120°C und Drücken bis 1,0 MPa, vorzugsweise von 0,2 bis 1,0 MPa erfolgt.

4. Verfahren nach einem oder mehreren der Ansprüche 1 bis 3, dadurch gekennzeichnet, daß die Extraktion mit Wasser und/oder mit der Lösung des komplexbildenden Reagens in mehreren Stufen erfolgt.

5. Verfahren nach einem oder mehreren der Ansprüche 1, 3 und 4, dadurch gekennzeichnet, daß die komplexbildenden Reagenzien Verbindungen sind, die zur Ausbildung von Koordinationsbindungen mit dem Rhodium befähigte Stickstoff- bzw. Phosphoratome enthalten.

6. Verfahren nach Anspruch 5, dadurch gekennzeichnet, daß die komplexbildenden Reagenzien Iminodiessigsäure, Nitrilotriessigsäure, Ethylendiamintetraessigsäure, phenylphosphintrisulfonate oder Triphenylphosphindisulfonate sind.

7. Verfahren nach einem oder mehreren der Ansprüche 1 und 3 bis 6, dadurch gekennzeichnet, daß je mol Rhodium mindestens 5 mol eines einzähnigen bzw. $\frac{5}{n}$ mol eines n-zähnigen, vorzugsweise 20 bis 40 mol eines einzähnigen bzw. $\frac{20}{n}$ bis $\frac{40}{n}$ mol eines n-zähnigen Komplexbildner angewendet werden.

8. Verfahren nach einem oder mehreren der Ansprüche 1 und 3 bis 7, dadurch gekennzeichnet, daß die Lösung des Komplexbildners 0,5 bis 25 Gew.-% Komplexbildner, bezogen auf die Lösung, enthält.

## Claims

1. A process for the recovery of rhodium which is contained in the distillation residue of products of the oxo synthesis, if appropriate bonded as a complex, by treatment of the residue with oxygen or an oxygen-containing gas at 60 to 120°C, under normal pressure or under pressure in the presence of a $C_2$- to $C_5$-monocarboxylic acid and of the alkali metal salt of a $C_2$- to $C_5$-monocarboxylic acid and subsequent extraction of the rhodium present as a water-soluble compound, which comprises extracting the residue with the aqueous solution of a reagent which complexes with rhodium, if appropriate after it has already been extracted beforehand with water by itself.

2. The process as claimed in claim 1, wherein the extraction with the solution of the complexing reagent is carried out at temperatures of 20 to 120°C and under pressures of up to 1.0 MPa, preferably from 0.2 to 1.0 MPa.

3. The process as claimed in claim 1, wherein the extraction is carried out with water at temperatures from 20 to 120°C and under pressures of up to 1.0 MPa, preferably from 0.2 to 1.0 MPa.

4. The process as claimed in one or more of claims 1 to 3, wherein the extraction is carried out with water and/or with the solution of the complexing reagent in several stages.

5. The process as claimed in one or more of claims 1, 3 and 4, wherein the complexing reagent is a compound which contains nitrogen or phosphorus atoms capable of forming coordination bonds with the rhodium.

6. The process as claimed in claim 5, wherein the complexing reagent is iminodiacetic acid, nitrilotriacetic acid, ethylenediaminetetraacetic acid, a triphenylphosphinetrisulfonate or a triphenylphosphinedisulfonate.

7. The process as claimed in one or more of claims 1 and 3 to 6, wherein at least 5 mol of a monodentate or $\frac{5}{n}$ mol of an n-dentate, preferably 20 to 40 mol of a monodentate or $\frac{20}{n}$ to $\frac{40}{n}$ mol of an n-dentate, complexing agent are used per mol of rhodium.

8. The process as claimed in one or more of claims 1 and 3 to 7, wherein the solution of the complexing agent contains 0.5 to 25% by weight of the complexing agent, based on the solution.

## Revendications

1. Procédé de récupération du rhodium qui est contenu dans le résidu de distillation de produits d'oxosynthèse, le cas échéant sous la forme d'une liaison complexe, en traitant le résidu avec de l'oxygène ou avec un gaz contenant de l'oxygène entre 60 et 120°C, sous pression ou non, en présence d'un acide monocarboxylique en $C_2$-$C_5$ et du sel alcalin d'un acide monocarboxylique en $C_2$-$C_5$, suivi d'une extraction du rhodium présent sous la forme d'un composé hydrosoluble, caractérisé en ce que le résidu est extrait avec la solution aqueuse d'un réactif formant un complexe avec le rhodium, le cas échéant après avoir subi auparavant une extraction avec de l'eau uniquement.

2. Procédé selon la revendication 1, caractérisé en ce que l'extraction avec la solution de réactif complexant se fait à des températures comprises entre 20 et 120°C et à des pressions allant jusqu'à 1,0 MPa, de préférence comprises entre 0,2 et 1,0 MPa.

3. Procédé selon la revendication 1, caractérisé en ce que l'extraction à l'eau se fait à des températures comprises entre 20 et 120°C et à des pressions allant jusqu'à 1,0 MPa, de préférence comprises entre 0,2 et 1,0 MPa.

4. Procédé selon l'une ou plusieurs des revendications 1 à 3, caractérisé en ce que l'extraction à l'eau et/ou avec la solution de réactif complexant se fait en plusieurs étapes.

5. Procédé selon l'une ou plusieurs des revendications 1, 3 et 4, caractérisé en ce que les réactifs complexants sont des composés qui possèdent des atomes d'azote ou de phosphore aptes à former des liaisons de coordination avec le rhodium.

6. Procédé selon la revendication 5, caractérisé en ce que les réactifs complexants sont l'acide iminodiacétique, l'acide nitrilotriacétique, l'acide éthylène diamine tétracétique, des triphénylphosphine trisulfonates ou des triphénylphosphine disulfonates.

7. Procédé selon l'une ou plusieurs des revendications 1 et 3 à 6, caractérisé en ce que l'on utilise pour une mole de rhodium au moins 5 moles d'un agent complexant de coordinance unité ou bien 5/n moles d'un agent complexant de coordinance n, de préférence de 20 à 40 moles d'un agent complexant de coordinance unité ou bien de 20/n à 40/n moles d'un agent complexant de coordinance n.

8. Procédé selon l'une ou plusieurs des revendications 1 et 3 à 7, caractérisé en ce que la solution d'agent complexant contient de 0,5 % à 25 % en poids d'agent complexant, ramené au poids de la solution.